# EUROPEAN PATENT SPECIFICATION

(11) **EP 4 009 983 B1**
(45) Date of publication and mention of the grant of the patent: **12.06.2024**
(21) Application number: 20761900.8
(22) Date of filing: 03.08.2020
(51) Int. Cl.: A61K 31/593, A61P 1/00, A61P 3/02, A61P 37/00, A23L 33/155, A61K 9/08, A61K 47/44

(54) **CHOLECALCIFEROL FOR USE IN THE TREATMENT OF CELIAC DISEASE**
CHOLECALCIFEROL ZUR VERWENDUNG BEI DER BEHANDLUNG VON ZÖLIAKIE
CHOLÉCALCIFÉROL DESTINÉ À ÊTRE UTILISÉ DANS LE TRAITEMENT DE LA MALADIE COELIAQUE

(30) Priority: 09.08.2019 IT 201900014550
(43) Date of publication of application: 15.06.2022
(73) Proprietor: Abiogen Pharma SpA, 56121 Ospedaletto (PISA) (IT)
(72) Inventor: TRASCIATTI, Silvia, 56019 Vecchiano (PI) (IT)
(74) Representative: Villa, Livia
(86) International application number: PCT/IB2020/057316
(87) International publication number: WO 2021/028772

(56) References cited:
- US-B1- 8 128 971
- LO ET AL: "Vitamin D absorption in healthy subjects and in patients with intestinal malabsorption syndromes.", AMERICAN JOURNAL OF CLINICAL NUTRITION, vol. 42, no. 4, 1 October 1985 (1985-10-01), pages 644-649, XP055052843, ISSN: 0002-9165

## Description

### FIELD OF THE INVENTION

The present invention relates to the use of cholecalciferol as an active agent in the treatment of celiac disease.

### STATE OF THE ART

Celiac disease is the most common chronic autoimmune enteropathy present in Western populations. This pathology is triggered by the ingestion of gluten in genetically predisposed subjects.

Gluten, a high molecular weight protein complex present in the endosperm of the kernels of cereals, including wheat, barley and rye, is stored in the seeds to ensure a stable supply of nutrients to support germination and development of young plants. Gluten-based cereals, which represent the most important crops in the world, are normally used for the production of food products such as pasta, bread, and various bakery and pastry products. The viscoelastic and stabilizing properties of gluten have in fact favoured its use as an additive in cooking operations in industrial ovens; in addition, gluten provides food with greater palatability, due to the creation of disulphide bonds which in combination with atmospheric oxygen and nitrogen alter the properties of the dough.

Gluten is a compound of two classes of proteins, glutenins and prolamins.

Prolamine is the protein fraction responsible for the toxic effect for celiacs.

The prolamin of wheat is called gliadin and constitutes about 50% of the gluten content. Similar proteins, with the same effect on celiac disease, are also found in barley (hordein), rye (secalin), emmer, spelled, kamut, triticale and oats (avenin).

Pepsin-trypsin resistant gliadin (PT-G) is the undigested fragment of gliadin that contributes to the pathogenesis of celiac disease by altering the tight intercellular junctions (TJs) and causing the intestinal barrier function to decrease.

Even very small amounts of gluten can cause problems. If eating habits are not changed, the intestinal villi regress and the damaged mucous membrane of the small intestine is no longer able to sufficiently metabolise nutrients, resulting in malnutrition.

In fact, gluten intolerance generates serious damage to the intestinal mucosa such as atrophy of the intestinal villi.

Celiac disease manifests itself in very different ways. Gastrointestinal disorders, such as diarrhoea, abdominal bloating, abdominal pain, nausea and vomiting often occur, but not always.

Other general symptoms can also be indicators of possible celiac disease. These include, for example, weight and energy loss, loss of appetite, iron deficiency with anaemia, osteoporosis, unsatisfied pregnancy desires or spontaneous abortions, vitamin and/or mineral deficiency.

In children, celiac disease develops in the first years of life, often already after weaning when they switch from breast milk to foods containing gluten. If it is not discovered in time, children could suffer from growth and development disorders. Children with celiac disease often have a thin constitution, are easy to cry and very sensitive.

Unfortunately, there are still no pharmacological therapies able to treat celiac disease and no drug on the market with this indication of use.

In the literature there are some preliminary studies both *in vivo* and *in vitro* that describe the possible positive effects on the intestinal mucosal barrier towards PT-G by calcitriol, which seems to have a protective action against TJs (Dong et Al. , Dig. Dis. Sci. (2018), 63: 92-104). But the same study concludes that further investigations and clinical studies are needed to determine the effective usability of calcitriol in the treatment of celiac disease. US patent US 8 128 971 B1 discloses a composition for treating celiac disease comprising L-Carnosine, Aronia Berry, Vitamin D3, Folic Acid, RNA, DNA, *Pulsitilla,* and *Lycopodium Clavatiu,* which is postulated to stimulate regrowth of villi which would then be able to more efficiently absorb nutrients. However, said patent does not provide any experimental evidence that Vitamin D3 (cholecalciferol) is an active agent effective to treat celiac disease.

In relation to clinical studies, a phase II clinical study has recently been launched in the United States, to verify the possibility of using latiglutenase, a mixture of two specific recombinant gluten proteases, capable of degrading gluten as a treatment for celiac disease into smaller physiologically irrelevant fragments. The drug, of biological type, would be administered in oral form, and would be associated with a gluten-free diet.

The gluten-free diet, carried out rigorously and for a lifetime, is, in fact, and unfortunately, to date, still the only therapy that guarantees the celiac a perfect state of health.

The need is therefore strongly felt to develop drugs that are able to treat celiac disease, or at least to counteract the harmful effects caused by the same.

The object of the present invention is therefore to find an effective remedy for the treatment of celiac disease, which is also well tolerated by the body.

### SUMMARY OF THE INVENTION

This object has been achieved by cholecalciferol as an active agent for use in the therapeutic treatment of celiac disease.

In another aspect, cholecalciferol as an active agent for use in the treatment of celiac disease is administered in a therapeutically effective amount for the reduction of intestinal villous atrophy.

In a further aspect, cholecalciferol as an active agent for use in the treatment of celiac disease is administered in a therapeutically effective amount for the protection and regeneration of the intestinal epithelium.

In a further aspect, the present invention relates to a pharmaceutical composition or a food supplement comprising cholecalciferol as an active agent, and at least one suitable excipient, for use in the treatment of celiac disease.

In a further aspect, the present invention relates to a food for celiacs comprising cholecalciferol as an active agent for use in the treatment of celiac disease, and at least one suitable food ingredient.

### DETAILED DESCRIPTION OF THE INVENTION

The present invention is defined by the claims. Any subject-matter falling outside the scope of the claims is provided for information only. Methods of treatment of the human body by therapy, referred to in this description, are not part of the present invention as such, but are described here in relation to compounds and pharmaceutical or food compositions for use in said methods of treatment of the human body by therapy, according to the present invention.

The invention concerns cholecalciferol as an active agent for use in the treatment of celiac disease.

Cholecalciferol, or vitamin D3, is the natural compound of vitamin D, of animal / human origin. It is a prohormone, precursor of the two hydroxylated forms [25OHD and 1,25(OH)2D] of vitamin D and therefore needs to undergo two natural hydroxylation processes to transform into its metabolically active form.

For the purposes of the present invention, the term "cholecalciferol" is understood to include all optical isomers, geometric isomers and stereoisomers, as well as their mixtures, such as mixtures of enantiomers, racemic mixtures and mixtures of diastereoisomers, as well as all their forms polymorphic, both amorphous and crystalline, and co-crystalline, as well as anhydrous, hydrated and solvated forms, pharmaceutically acceptable salts, and mixtures thereof.

As will also be clear from the examples below, cholecalciferol has been shown to be an active agent that can be used in the treatment of celiac disease.

In particular, cholecalciferol has shown a marked activity in reducing the atrophy of the intestinal villi. Cholecalciferol can therefore be effectively used as an active agent in reducing intestinal villous atrophy. The present invention therefore also concerns the use of a therapeutically effective amount of cholecalciferol as an active agent in reducing intestinal villous atrophy. The Examples provided below have in fact shown that the villi of animals treated with cholecalciferol were longer than those of untreated celiac controls. Since the decreased villous length is a sign of villous atrophy and celiac enteropathy, it can be said that the results of the histomorphometric analysis were decidedly positive, being the villi of the animals treated with cholecalciferol longer than those of the untreated celiac controls; moreover, the increase in the length of the villi was shown to be proportional to the dose of cholecalciferol administered.

In addition, cholecalciferol has also shown a marked activity in the protection and regeneration of the intestinal epithelium, so cholecalciferol can also be effectively used as an active agent in the protection and regeneration of the intestinal epithelium. The present invention therefore also concerns the use of a therapeutically effective amount of cholecalciferol as an active agent in the protection and regeneration of the intestinal epithelium.

In some embodiments, the present invention relates to cholecalciferol as an active agent for use in the treatment of celiac disease, wherein said treatment comprises the administration of a therapeutically effective amount of cholecalciferol for the reduction of intestinal villous atrophy.

In other embodiments, the present invention relates to cholecalciferol as an active agent for use in the treatment of celiac disease, wherein said treatment comprises the administration of a therapeutically effective amount of cholecalciferol for the protection and regeneration of the intestinal epithelium.

In further embodiments, the present invention relates to cholecalciferol as an active agent in the treatment of celiac disease, wherein said treatment comprises the administration of a therapeutically effective amount of cholecalciferol for the reduction of intestinal villous atrophy and the protection and regeneration of the intestinal epithelium.

Preferably, cholecalciferol is to be administered in a dose of 0.4 µg/kg/day to 11 µg/kg/day, more preferably 0.8 µg/kg/day to 11 µg/kg/day, and even more preferably 4 µg/kg/day to 11 µg/kg/day.

In other words, preferably cholecalciferol is to be administered in a dose of 16 IU/kg/day to 430 IU/kg/day, more preferably 30 IU/kg/day to 430 IU/kg/day, and even more preferably 160 IU/kg/day to 430 IU/kg/day.

Preferably, the cholecalciferol is to be administered via oral, injective or subcutaneous route, more preferably via oral route. With the exception of particular clinical conditions (e.g. malabsorption syndromes), the oral route of administration is preferable, as it is superior in terms of efficacy in increasing serum 25OHD compared to the intramuscular formulation.

In another aspect, the present invention relates to a pharmaceutical composition or a food supplement, comprising cholecalciferol as an active agent, and at least one suitable excipient, for use in the treatment of celiac disease.

In some embodiments, the present invention relates to a pharmaceutical composition or a food supplement, comprising cholecalciferol as an active agent, and at least one suitable excipient, for use in the treatment of celiac disease, wherein said treatment comprises the administration of a pharmaceutical composition or dietary supplement comprising a therapeutically effective amount of cholecalciferol for the reduction of intestinal villous atrophy.

In other embodiments, the present invention relates to a pharmaceutical composition or a food supplement, comprising cholecalciferol as an active agent, and at least one suitable excipient, for use in the treatment of celiac disease, wherein said treatment comprises the administration of a pharmaceutical composition or dietary supplement comprising a therapeutically effective amount of cholecalciferol for the protection and regeneration of the intestinal epithelium.

In further embodiments, the present invention relates to a pharmaceutical composition or a food supplement, comprising cholecalciferol as an active agent, and at least one suitable excipient, for use in the treatment of celiac disease, wherein said treatment comprises the administration of a pharmaceutical composition or dietary supplement comprising a therapeutically effective amount of cholecalciferol for the reduction of intestinal villous atrophy and the protection and regeneration of the intestinal epithelium.

Said pharmaceutical composition or dietary supplement can advantageously also comprise at least one probiotic.

The pharmaceutical composition, as well as the food supplement of the invention, can be administered via oral, injective or subcutaneous route.

In a preferred embodiment, said pharmaceutical composition or dietary supplement is to be administered via oral route.

When the pharmaceutical composition or dietary supplement is to be administered via oral route, it is preferably in the form of an orodispersible solid preparation, gel, capsule, tablet, powder, granulate, solution, suspension, emulsion or tincture.

The pharmaceutical composition or food supplement of the invention to be administered via oral route in the form of an orodispersible solid preparation, gel, capsule, tablet, powder, granulate, solution, suspension, emulsion or tincture, is preferably administered in a dose so as to provide 0.4 µg/kg/day to 11 µg/kg/day of cholecalciferol, preferably 0.8 µg/kg/day to 11 µg/kg/day, even more preferably 4 µg/kg/day to 11 µg/kg/day.

In other words, said pharmaceutical composition or food supplement of the invention to be administered via oral route in the form of an orodispersible solid preparation, gel, capsule, tablet, powder, granulate, solution, suspension, emulsion or tincture, is preferably administered in a dose so as to provide 16 IU/kg/day to 430 IU/kg/day of cholecalciferol, preferably 30 IU/kg/day to 430 IU/kg/day, even more preferably 160 IU/kg/day to 430 IU/kg/day.

In preferred embodiments, the pharmaceutical composition or food supplement of the invention to be administered via oral route, in the form of a solution, suspension, emulsion, gel or tincture, comprises cholecalciferol in a concentration of 10,000 IU/mL to 20,000 IU/mL.

Said pharmaceutical composition or food supplement of the invention can advantageously be in the form of a unit dose.

Said pharmaceutical composition or food supplement of the invention, also in the form of a unit dose, also comprises at least one suitable excipient. The term "excipient" means a compound or a mixture of compounds suitable for pharmaceutical or food use, respectively. For example, an excipient for use in a pharmaceutical or food formulation generally must not cause an adverse response in a subject, nor must it significantly inhibit the efficacy of the cholecalciferol contained therein.

Suitable excipients are acidifiers, acidity regulators, anti-caking agents, antioxidants, bulking agents, strength agents, gelling agents, coating agents, modified starches, sequestrants, thickeners, sweeteners, thinners, disaggregants, glidants, dyes, binders, lubricants, stabilizers, adsorbents, humectants, flavours, film-forming substances, emulsifiers, wetting agents, release retardants and mixtures thereof.

Preferably, said excipients are olive oil, mineral oil, liquid paraffin, white petrolatum, polyoxyethylene, emulsifying wax, stearyl alcohol, isostearyl alcohol, cetylstearyl alcohol, stearic acid, glyceryl stearate, sodium lauryl sarcosinate, glycerine, diethylene glycolmonoethyl ether, polyethylene glycols, polyethylene glycol stearates, carbopol, carbomers, Poloxamer 407, Macrogol 400, purified bentonite, myristyl propionate, dimethicone, titanium dioxide, anionic, cationic and non-ionic surfactants, water, potassium sorbate, sodium benzoate, ε-polylysine, sucralose, maltodextrin, citric acid, sodium carbonate, calcium carbonate, magnesium carbonate, magnesium stearate, natural starch, partially hydrolysed starch, modified starch, lactose, calcium phosphate, calcium carbonate, calcium sulphate, polyvinylpyrrolidone, silica, colloidal silica, precipitated silica, magnesium silicates, aluminum silicates, sodium lauryl sulphate, magnesium lauryl sulphate, methacrylate copolymers, sodium dehydroacetate, xanthan gum, guar gum, tara gum, carob gum, fenugreek gum, Arabic gum, alginic acid, sodium alginate, propylene glycol alginate, sodium croscarmellose, polyvinylpolypyrrolidone, glyceryl behenate, indigo carmine, cellulose, modified cellulose, calcium carboxymethylcellulose, sodium carboxymethylcellulose, microcrystalline cellulose, ethyl cellulose, gelatine, hydroxyethyl cellulose, hydroxypropyl cellulose, polydextrose, carrageenan, methylcellulose, sucrose, sucrose gum esters, sorbitol, xylitol, dextrose, fructose, maltitol, gum tragacanth, pectin, agar, carboxypolymethylene, hydroxypropyl methylcellulose, tragacanth, mannitol, or a mixture thereof.

In some embodiments, the pharmaceutical composition or the food supplement of the invention consists essentially of cholecalciferol as an active agent and at least one suitable excipient. The expression "consists essentially of" means that cholecalciferol is the only active ingredient in the treatment of celiac disease to be present in the composition or supplement, whereas any additional components, as said at least one probiotic, or excipients do not interfere with its action. It should be understood that all the aspects identified above as preferred and advantageous for the use of cholecalciferol, the composition or the food supplement, are to be considered likewise preferred and advantageous also for these embodiments.

In other embodiments, the pharmaceutical composition or food supplement of the invention consists of cholecalciferol as an active agent, at least one suitable excipient, and optionally at least one probiotic.

In a further preferred embodiment of the present invention, said composition is a food for celiacs comprising cholecalciferol as an active agent for the use described above, and at least one suitable food ingredient.

It has been surprisingly found that the presence of cholecalciferol allows celiacs to feed themselves, without necessarily resorting to gluten-free products. Therefore, advantageously, according to the present invention, the term "food for celiacs" means any edible product, with or without gluten, intended for both human and animal feeding, including baked goods, alcoholic beverages, non-alcoholic beverages, energy, diet bars, condiments, the so-called "breakfast cereals", fresh pasta, dry pasta, milk and its derivatives (such as yogurt and ice cream), fruit juices, sweets, as well as animal feed.

Preferably, said food for celiacs is a baked product, such as pasta, bread, sweet snack, savoury snack, biscuit, breadstick, cracker, dessert, savoury pie, and in general any type of cooked dough comprising flour, with or without gluten, more preferably gluten-free. Preferably, said food for celiacs contains cholecalciferol in an amount of 200 to 2,000 IU per serving.

In preferred embodiments, when said food for celiacs is a baked product, it contains cholecalciferol in an amount of 1 to 5 IU/g.

Said food for celiacs can be consumed with the aim of contributing to the reduction of intestinal villous atrophy and the protection and regeneration of intestinal villi.

Said food for celiacs can advantageously also comprise at least one probiotic, as described above.

All the pharmaceutical compositions or food supplements or celiac foods described above can be prepared by methods known in the art.

It should be understood that all the possible combinations of the preferred aspects of cholecalciferol, of the products containing the same and the respective uses, as indicated above, are also described, and therefore likewise preferred.

It should also be understood that all the aspects identified as preferred and advantageous for cholecalciferol and its products are to be considered likewise preferred and advantageous also for their preparation and uses.

The efficacy of cholecalciferol as an active agent in the treatment of celiac disease will also be clear from the examples given below.

Without wishing to be bound by any theory, it is believed that cholecalciferol is beneficial in the context of the treatment of celiac disease also because it can influence the gut microbiota profile through the regulation of anti-microbial peptides which, in turn, regulate the intestinal microbial community, ensuring homeostasis of beneficial bacterial strains. This action would be mediated, at the molecular level, by the vitamin D receptor (VDR).

Furthermore, cholecalciferol is normally stored in adipose tissue, where it creates deposits from which it is slowly released. Precisely for this reason, it has a rather short blood half-life (T_{1/2} estimated at 19-25 hours), while its functional half-life (several weeks) is much longer (related to slow release). The high functional half-life (slow release by adipose tissue) makes cholecalciferol an extremely flexible and adaptable active agent in clinical practice, allowing even intermittent administration regimens.

Below are working examples of the present invention provided for illustrative and nonlimiting purposes.

### EXAMPLES

### Example 1 - Evaluation of the efficacy of repeated oral administration of cholecalciferol in an in two mouse model of celiac disease (NOD mice)

For the study, the NOD (Non Obese Diabetic) mouse was chosen as an experimental model which, when fed with a standard diet containing gluten, is able to spontaneously develop enteropathy and autoimmune disorders, with alterations of the small intestine similar to those observed. in human celiac disease.

103 female NOD mice aged four weeks were then placed in two different enclosure rooms (one for mice intended to follow a standard diet and one for mice intended for a gluten-free diet) and allowed to acclimate for a period of at least seven days.

They were then randomly assigned to seven different experimental groups. The development of enteropathy was achieved by feeding NOD mice with a standard diet containing gluten and administering gliadin orally with a dosage of 10 µg/kg for ten consecutive days.

The seven experimental groups are represented in the following table 1.

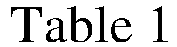

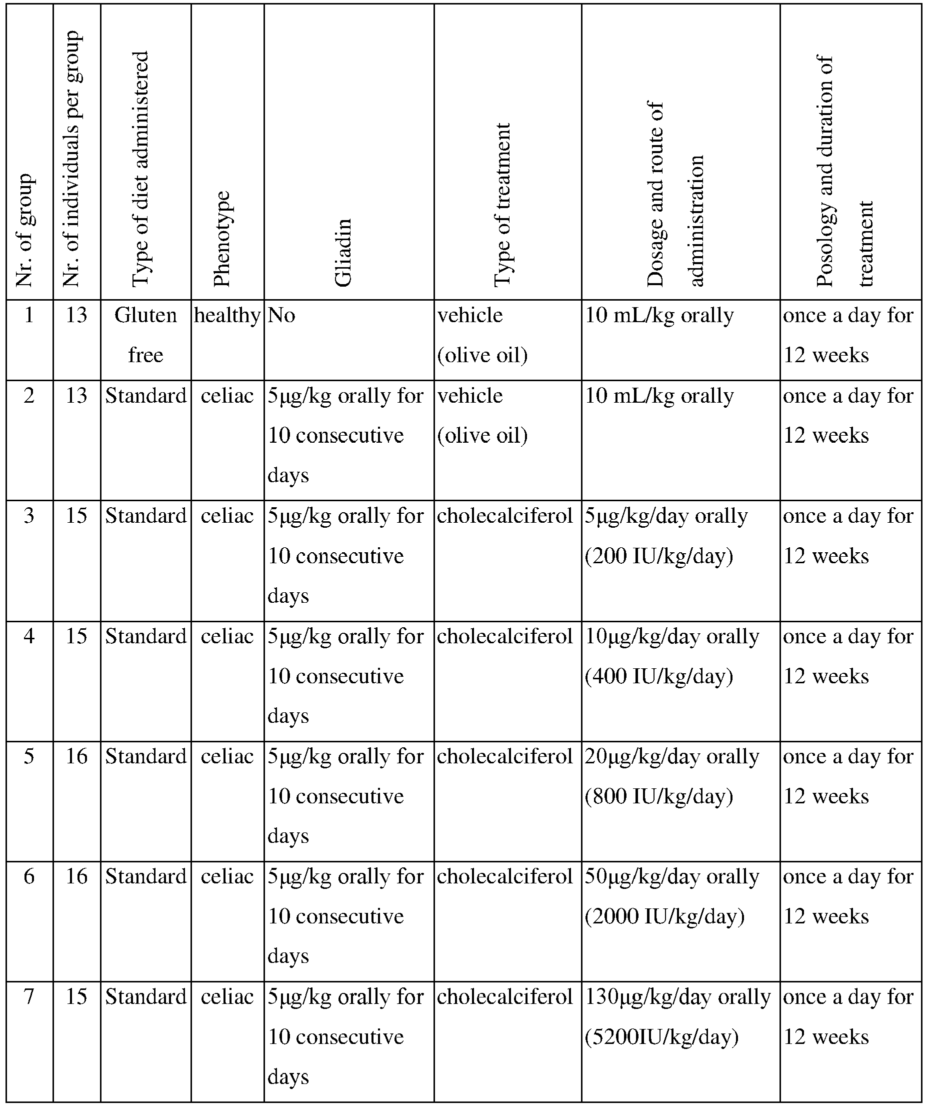

At the end of the treatment period the mice were sacrificed. A macroscopic examination was performed and the small intestine was removed from each mouse to be subjected to microscopic examinations.

In particular, histopathological and histomorphometric examinations were carried out on sections of intestine with a thickness of 5-7 µm placed in paraffin and stained with hematoxylin-eosin.

The scoring system described in Marsh et al, Gut 1990 Jan; 31 (1): 111-4 was used, to allow comparison between groups with different degrees of inflammatory infiltration and type of lesions observed under the microscope, which provides for the following classification:
- lesion type 0: normal intestinal mucosa;
- lesion type 1 (infiltrative): normal morphology and architecture of the villi, higher number of intraepithelial lymphocytes;
- lesion type 2 (hyperplastic): normal morphology and architecture of the villi, higher number of intraepithelial lymphocytes, hyperplasia of the glandular elements;
- lesion type 3 (infiltrative): different degrees of atrophy of the villi with hypertrophy of the glandular crypts, superficial enterocytes with reduced height, higher number of intraepithelial lymphocytes.

Fifteen histomorphometric measurements of the height of the villi were then carried out from each of the five small intestine sections evaluated for each mouse.

The results of the microscopic histopathological evaluation are summarized in the following table 2, which contains the four degrees of lesion previously described and the number of animals of each group presenting the various types of lesions.

**Table 2**

| | Lesion type 0 | Lesion type 1 | Lesion type 2 | Lesion type 3 |
|---|---|---|---|---|
| Control (n=13) | 7 | 5 | 1 | 0 |
| Control pathology (n=13) | 0 | 1 | 7 | 5 |
| Cholecalciferol 5µg/kg (n=15) | 0 | 2 | 13 | 0 |
| Cholecalciferol 10 µg/kg (n=15) | 0 | 9 | 4 | 2 |
| Cholecalciferol 20 µg/kg (n=16) | 0 | 7 | 4 | 5 |
| Cholecalciferol 50 µg/kg (n=16) | 3 | 3 | 9 | 1 |
| Cholecalciferol 130 µg/kg (n=15) | 3 | 3 | 6 | 3 |

The most severe histological representation for the small intestine was observed in the group of untreated celiacs, wherein 5 out of 13 animals showed lesions of the greatest extent.

On the other hand, at all tested doses of cholecalciferol, an overall improvement in the general depiction of the lesions was observed in the treated mice compared to the untreated celiac mice. This depiction significantly improved when the treatment was conducted at the highest cholecalciferol doses, i.e. when the mice were treated with repeated administrations of 50 and 130 µg/kg of cholecalciferol.

The results of the histomorphometric microscopic evaluation of the villus length are shown in the following table 3.

**Table 3**

| | Villus length (x10µm) |
|---|---|
| Control (n=13) | 78.0 |
| Control pathology (n=13) | 61.3 |
| Cholecalciferol 5µg/kg (n=15) | 65.7 |
| Cholecalciferol 10 µg/kg (n=15) | 71.2 |
| Cholecalciferol 20 µg/kg (n=16) | 76.1 |
| Cholecalciferol 50 µg/kg (n=16) | 75.0 |
| Cholecalciferol 130 µg/kg (n=15) | 78.8 |

As evident from the data indicated in Table 3 above, the villi of the animals treated with cholecalciferol proved to be longer than those of the untreated celiac controls.

Since the decreased villous length is a sign of villous atrophy and celiac enteropathy, it can be said that the results of the histomorphometric analysis were decidedly positive, with the villi of the animals treated with cholecalciferol longer than those of the untreated celiac controls, and with a length that proved to be the greater the higher the doses of cholecalciferol administered, thus showing a dose-dependent relationship between the increase in the length of the villi and the dose of cholecalciferol administered.

### Example 2 - Analysis of the expression of some proteins in the mucosa of the small intestine of NOD mice by immunohistochemistry (IHC)

Small intestine samples were taken from mice belonging to experimental groups 2 (pathology control) and 7 (cholecalciferol 130 µg/kg) of the experiment described in Example 1 and were processed to evaluate by immunohistochemistry the expression of three proteins: the receptor for vitamin D (VDR), the T-cell marker (CD3) which indicates lymphocyte infiltration and the protein of Zonula Occludens 1 or Zonulin-1 (ZO-1) which is usually released in serum and tissue following destruction of the intestinal epithelial barrier, in particular of the intercellular tight junctions (TJs).

The samples were washed in xylene, hydrated and washed in water with solutions of ethanol and water to scale. The recovery of the antigen was carried out in sodium citrate buffer using the standard "pressure cooker" protocol. Once the antigen recovery was completed, the samples were placed at room temperature for 10 'and washed twice for 2' in PBS tween (phosphate buffer saline with tween). Non-specific sites were blocked with 200 µL of T20 starting block.

They were then dispensed on each slide, either 200 µL of solution comprising the primary antibody of interest, or 200 µL of the respective negative control. The slides were then left to incubate overnight in a humid chamber with PBS at 4°C.

After incubation, repeated washes were carried out in wash buffer at room temperature, after which the endogenous peroxidase was blocked with a 0.3% hydrogen peroxide buffer.

Subsequently, repeated washes were carried out again in wash buffer at room temperature and the secondary antibody was added, which was left to incubate for 1 hour at room temperature.

Repeated washings were then carried out again in wash buffer at room temperature. Subsequently, the DAB solution (3',3'-Diaminobenzidine) was added, which was then blocked by washing at room temperature.

Finally, counterstaining with Meyer's hematoxylin was carried out and the reaction was stopped by washing in water.

The slides were then mounted with two drops of aqueous mounting medium and allowed to dry.

The atrophy of the intestinal mucosa was evaluated according to the Corazza-Villanacci classification.

Antibody labelling in villi, crypts and goblet cells was evaluated.

### Results

### VDR

A reduction in atrophy in the villi of the group of animals treated with cholecalciferol compared to the vehicle treated control was detected.

The villi were more marked, therefore with a greater expression of VDR, in the group treated with cholecalciferol and the same phenomenon was observed in the crypts. As for the goblet cells, the group treated with the active showed a smaller number of marked cells, albeit with a greater intensity of marking.

### CD3

This protein was less expressed in the group treated with cholecalciferol, while the villi and crypts of the same group were unlabeled. This means that there was no intraepithelial lymphocyte cell infiltrate, indicating a lower immune response, which underlies the pathogenesis of celiac disease.

The goblet cells of the group treated with the active also showed a reduction in the signal compared to the control and also a reduction in the intensity of the marking.

### ZO-1

Atrophy of the villi was less marked in the group treated with cholecalciferol.

The villi and crypts were more marked in the group treated with the vehicle.

### Example 3 - Pharmaceutical composition comprising cholecalciferol for oral use in the form of a solution

In order to make pharmaceutical compositions in the form of a solution for oral use, the cholecalciferol was mixed with suitable excipients.

The compositions made contained a unit dosage of about 50,000 IU and 25,000 IU per bottle.

The quantities of excipients contained in each bottle are indicated in the following tables 4a and 4b, respectively.

**Table 4a**

| Component | Unitary amount |
|---|---|
| Cholecalciferol | 50,000 UI |
| Refined olive oil | Volume balance to 2.5 mL |

**Table 4b**

| Component | Unitary amount |
|---|---|
| Cholecalciferol | 25,000 UI |
| Refined olive oil | Volume balance to 2.5 mL |

After having introduced refined olive oil into a dissolution tank heated to 40°C ± 3°C (checking that the temperature never exceeded 45°C), cholecalciferol was added under nitrogen, stirring until complete dissolution (at least 60 minutes). The mixture was then cooled to about 27°C (± 3°C) and filtered under nitrogen at the maximum pressure of 0.8 atm in the tank.

The filtered solution was finally automatically dosed inside amber III type bottles and sealed inside them.

### Example 4- Pharmaceutical compositions comprising cholecalciferol for oral use in the form of a hard gelatin capsule

In order to make a pharmaceutical composition in the form of a hard capsule for oral use, cholecalciferol was mixed with suitable excipients.

The compositions made contained a unit dosage of about 50,000 IU and 25,000 IU per capsule.

The amounts of excipients contained in each capsule are indicated in the following tables 5a and 5b, respectively.

**Table 5a**

| **Component** | **Unitary amount (mg)** |
|---|---|
| Cholecalciferol | 1.250 mg (equivalent to 50,000 UI) |
| Refined olive oil | 181.150 mg |

**Table 5b**

| **Component** | **Unitary amount (mg)** |
|---|---|
| Cholecalciferol | 0.625 mg (equivalent to 25,000 UI) |
| Refined olive oil | 181.775 mg |

Cholecalciferol was added to the refined olive oil in a dissolution tank, under nitrogen, leaving it stirred until completely dissolved (for at least 60 minutes).

The capsules were filled, still under nitrogen, by means of automatic machines, with 200µL of the cholecalciferol solution thus prepared.

Subsequently, the capsules were sealed with banding by means of a gelatine band and packaged in PVC / PVDC / Al blisters by means of an automatic blistering machine.

### Example 5 - Preparation of a food containing cholecalciferol

A focaccia containing cholecalciferol and suitable ingredients was prepared, as reported in table 6.

**Table 6**

| **Component** | **Amount** |
|---|---|
| Bread flour (with or without gluten) | 400g |
| Lukewarm water | 200 mL |
| Lukewarm milk | 120 mL |
| Brewer's yeast | 10 g |
| Extra virgin olive oil | 2 spoons |
| Cholecalciferol | 2,000 UI |
| Salt | Just enough |
| Chopped rosemary | Just enough |

In a container, the yeast was dissolved in water, which was then added to the flour and mixed.

Later, milk was added gradually and under stirring to the mixture.

A portion of olive oil (about a tablespoon) and salt was then added to the dough and continued stirring.

The dough was placed in a suitable non-stick container, spread and left to rise for about 2 hours.

The dough was then sprinkled with coarse salt and rosemary and cooked at about 220°C for 25 minutes.

At the end, the back of the focaccia was sprinkled with the remaining spoonful of olive oil, in which 2,000 IU of cholecalciferol had been dissolved.

### Conclusions

In conclusion, all the tests described above demonstrate that cholecalciferol is an active agent that can be used in the treatment of celiac disease.

In particular, cholecalciferol has shown a marked activity in reducing intestinal villous atrophy, therefore it can be effectively used as an active agent in reducing intestinal villous atrophy.

In addition, cholecalciferol has also shown a marked activity in the protection and regeneration of the intestinal epithelium, so it can also be effectively used as an active agent in the protection and regeneration of the intestinal epithelium.

## Claims

1. Cholecalciferol as an active agent for use in the treatment of celiac disease.

2. Cholecalciferol for the use of claim 1, wherein said treatment of celiac disease comprises the administration of a therapeutically effective amount of cholecalciferol for the reduction of intestinal villous atrophy.

3. Cholecalciferol for use of claim 1, wherein said treatment of celiac disease comprises the administration of a therapeutically effective amount of cholecalciferol for the protection and regeneration of the intestinal epithelium.

4. Cholecalciferol for the use of claim 1, wherein said treatment of celiac disease comprises the administration of a therapeutically effective amount of cholecalciferol for the reduction of intestinal villous atrophy and the protection and regeneration of the intestinal epithelium.

5. Cholecalciferol for the use of any one of claims 1-4, wherein cholecalciferol is to be administered in a dose of 0.4 µg/kg/day to 11 µg/kg/day, preferably 0.8 µg/kg/day to 11 µg/kg/day, more preferably 4 µg/kg/day to 11 µg/kg/day, or in a dose of 16 IU/kg/day to 430 lU/kg/day, preferably 30 IU/kg/day to 430 IU/kg/day, more preferably 160 IU/kg/day to 430 IU/kg/day.

6. Cholecalciferol for the use of any one of claims 1-5, wherein cholecalciferol is to be administered via oral, injective or subcutaneous route, preferably via oral route.

7. Pharmaceutical composition or food supplement comprising cholecalciferol as an active agent for use in the treatment of celiac disease according to any one of claims 1-6, and at least one suitable excipient.

8. The pharmaceutical composition or food supplement for use according to claim 7, wherein said pharmaceutical composition or food supplement is to be administered via oral route, and is in the form of a solid orodispersible preparation, gel, capsule, tablet, powder, granulate, solution, suspension, emulsion or tincture.

9. The pharmaceutical composition or food supplement for use according to claim 8, in the form of a solution, suspension, gel emulsion or tincture and comprising cholecalciferol in a concentration of 10,000 IU/mL to 20,000 IU/mL.

10. Food for celiacs comprising cholecalciferol as an active agent for use in the treatment of celiac disease according to any one of claims 1-6, and at least one suitable food ingredient.

11. The food for celiacs for use according to claim 10, wherein said food is an edible product intended for both human and animal consumption, selected from bakery products, alcoholic beverages, soft drinks, energy drinks, diet bars, condiments, the so-called "Breakfast cereals", fresh pasta, dry pasta, milk and its derivatives, fruit juices and sweets, and animal feed.

12. The food for celiacs for use according to claim 10 or 11, wherein cholecalciferol is present in an amount of 200 to 2,000 IU per portion, or in an amount of 1 to 5 IU/g of food.

13. The pharmaceutical composition or food supplement for use according to any one of claims 7-9, or the food for celiacs for use according to any one of claims 10-12, further comprising at least one probiotic.

## Patentansprüche

1. Cholecalciferol als ein Wirkstoff zur Verwendung bei der Behandlung von Zöliakie.

2. Cholecalciferol zur Verwendung nach Anspruch 1, wobei die Behandlung von Zöliakie die Verabreichung einer therapeutisch wirksamen Menge Cholecalciferol zur Verringerung der Darmzottenatrophie umfasst.

3. Cholecalciferol zur Verwendung nach Anspruch 1, wobei die Behandlung von Zöliakie die Verabreichung einer therapeutisch wirksamen Menge Cholecalciferol zum Schutz und zur Regeneration des Darmepithels umfasst.

4. Cholecalciferol zur Verwendung nach Anspruch 1, wobei die Behandlung von Zöliakie die Verabreichung einer therapeutisch wirksamen Menge Cholecalciferol zur Verringerung der Darmzottenatrophie und zum Schutz und zur Regeneration des Darmepithels umfasst.

5. Cholecalciferol zur Verwendung nach einem der Ansprüche 1-4, wobei Cholecalciferol in einer Dosis von 0,4 µg/kg/Tag bis 11 µg/kg/Tag, bevorzugt 0,8 µg/kg/Tag bis 11 µg/kg/Tag, besonders bevorzugt 4 µg/kg/Tag bis 11 µg/kg/Tag, oder in einer Dosis von 16 IE/kg/Tag bis 430 IE/kg/Tag, bevorzugt 30 IE/kg/Tag bis 430 IE/kg/Tag, besonders bevorzugt 160 IE/kg/Tag bis 430 IE/kg/Tag, zu verabreichen ist.

6. Cholecalciferol zur Verwendung nach einem der Ansprüche 1-5, wobei Cholecalciferol oral, mittels Injektion oder subkutan zu verabreichen ist, bevorzugt oral.

7. Pharmazeutische Zusammensetzung oder Nahrungsergänzungsmittel, umfassend Cholecalciferol als einen Wirkstoff zur Verwendung bei der Behandlung von Zöliakie nach einem der Ansprüche 1-6 und mindestens einen geeigneten Hilfsstoff.

8. Pharmazeutische Zusammensetzung oder Nahrungsergänzungsmittel zur Verwendung nach Anspruch 7, wobei die pharmazeutische Zusammensetzung oder das Nahrungsergänzungsmittel oral zu verabreichen ist und in der Form einer festen, in der Mundhöhle löslichen Zubereitung, eines Gels, einer Kapsel, einer Tablette, eines Pulvers, Granulats, einer Lösung, Suspension, Emulsion oder Tinktur vorliegt.

9. Pharmazeutische Zusammensetzung oder Nahrungsergänzungsmittel zur Verwendung nach Anspruch 8 in der Form einer Lösung, Suspension, Gelemulsion oder Tinktur und Cholecalciferol in einer Konzentration von 10.000 IE/ml bis 20.000 IE/ml umfassend.

10. Nahrungsmittel für Zöliakiebetroffene, umfassend Cholecalciferol als einen Wirkstoff zur Verwendung bei der Behandlung von Zöliakie nach einem der Ansprüche 1-6 und mindestens eine geeignete Nahrungsmittelzutat.

11. Nahrungsmittel für Zöliakiebetroffene zur Verwendung nach Anspruch 10, wobei das Nahrungsmittel ein essbares Produkt ist, das sowohl für menschlichen als auch tierischen Verzehr bestimmt ist, ausgewählt aus Backwaren, alkoholischen Getränken, Erfrischungsgetränken, Energydrinks, Diätriegeln, Würzmitteln, den sogenannten "Frühstückszerealien", frischen Teigwaren, trockenen Teigwaren, Milch und deren Derivaten, Fruchtsäften und Süßigkeiten und Tierfutter.

12. Nahrungsmittel für Zöliakiebetroffene zur Verwendung nach Anspruch 10 oder 11, wobei Cholecalciferol in einer Menge von 200 bis 2.000 IE pro Portion oder in einer Menge von 1 bis 5 IE/g des Nahrungsmittels vorhanden ist.

13. Pharmazeutische Zusammensetzung oder Nahrungsergänzungsmittel zur Verwendung nach einem der Ansprüche 7-9 oder das Nahrungsmittel für Zöliakiebetroffene zur Verwendung nach einem der Ansprüche 10-12, ferner mindestens ein Probiotikum umfassend.

## Revendications

1. Cholécalciférol en tant qu'agent actif destiné à être utilisé dans le traitement de la maladie coeliaque.

2. Cholécalciférol destiné à être utilisé selon la revendication 1, ledit traitement de la maladie coeliaque comprenant l'administration d'une quantité thérapeutiquement efficace de cholécalciférol pour la réduction de l'atrophie villositaire intestinale.

3. Cholécalciférol destiné à être utilisé selon la revendication 1, ledit traitement de la maladie coeliaque comprenant l'administration d'une quantité thérapeutiquement efficace de cholécalciférol pour la protection et la régénération de l'épithélium intestinal.

4. Cholécalciférol destiné à être utilisé selon la revendication 1, ledit traitement de la maladie coeliaque comprenant l'administration d'une quantité thérapeutiquement efficace de cholécalciférol pour la réduction de l'atrophie villositaire intestinale, et la protection et la régénération de l'épithélium intestinal.

5. Cholécalciférol destiné à être utilisé selon l'une quelconque des revendications 1 à 4, ledit cholécalciférol étant destiné à être administré à une dose de 0,4 µg/kg/jour à 11 µg/kg/jour, de préférence 0,8 µg/kg/jour à 11 µg/kg/jour, idéalement 4 µg/kg/jour à 11 µg/kg/jour, ou à une dose de 16 IU/kg/jour à 430 IU/kg/jour, de préférence 30 IU/kg/jour à 430 IU/kg/jour, idéalement 160 IU/kg/jour à 430 IU/kg/jour.

6. Cholécalciférol destiné à être utilisé selon l'une quelconque des revendications 1 à 5, ledit cholécalciférol devant être administré par voie orale, injectable ou sous-cutanée, de préférence par voie orale.

7. Composition pharmaceutique ou complément alimentaire comprenant le cholécalciférol en tant qu'agent actif destiné à être utilisé dans le traitement de la maladie coeliaque selon l'une quelconque des revendications 1 à 6, et au moins un excipient approprié.

8. Composition pharmaceutique ou complément alimentaire destiné à être utilisé selon la revendication 7, ladite composition pharmaceutique ou ledit complément alimentaire étant destiné à être administré par voie orale, et se présentant sous forme d'une préparation solide, d'un gel, d'une gélule, d'un comprimé, d'une poudre, d'un granulé, d'une solution, d'une suspension, d'une émulsion ou d'une teinture orodispersible.

9. Composition pharmaceutique ou complément alimentaire destiné à être utilisé selon la revendication 8, sous la forme d'une solution, d'une suspension, d'un gel, d'une émulsion ou d'une teinture et comprenant du cholécalciférol à une concentration de 10 000 IU/mL à 20 000 IU/mL.

10. Aliment pour personnes coeliaques comprenant le cholécalciférol en tant qu'agent actif destiné à être utilisé dans le traitement de la maladie coeliaque selon l'une quelconque des revendications 1 à 6, et au moins un ingrédient alimentaire approprié.

11. Aliment pour personnes coeliaques destiné à être utilisé selon la revendication 10, ledit aliment étant un produit comestible destiné à la consommation humaine et animale, choisi parmi les produits de boulangerie, les boissons alcooliques, les boissons gazeuses, les boissons énergétiques, les barres diététiques, les condiments, les céréales dites « de petit-déjeuner », les pâtes fraîches, les pâtes sèches, le lait et ses dérivés, les jus de fruit et les sucreries, et les aliments pour animaux.

12. Aliment pour personnes coeliaques destiné à être utilisé selon la revendication 10 ou 11, dans lequel le cholécalciférol est présent en une quantité de 200 à 2 000 IU par portion ou en une quantité de 1 à 5 IU/g d'aliment.

13. Composition pharmaceutique ou complément alimentaire destiné à être utilisé selon l'une quelconque des revendications 7 à 9, ou l'aliment pour personnes coeliaques destiné à être utilisé selon l'une quelconque des revendications 10 à 12, comprenant en outre au moins un probiotique.
